# EUROPEAN PATENT APPLICATION

(11) **EP 4 353 443 A1**
(43) Date of publication of application: **17.04.2024**
(21) Application number: 22820133.1
(22) Date of filing: 02.06.2022
(51) Int. Cl.: B29C 49/28, B29C 49/80

(54) **BLOW MOLDING MACHINE AND METHOD FOR DETERMINING PRODUCT DEFECT**

(30) Priority: 07.06.2021 JP 2021095079
(71) Applicant: Suntory Holdings Limited, Osaka 530-8203 (JP)
(72) Inventor: ASAMI, Taro, Tokyo 135-8631 (JP); WATANABE, Takeshi, Hokuto-shi, Yamanashi 408-0316 (JP); WATANABE, Miyuki, Hokuto-shi, Yamanashi 408-0316 (JP)
(74) Representative: Vossius & Partner Patentanwälte Rechtsanwälte mbB
(86) International application number: PCT/JP2022/022473
(87) International publication number: WO 2022/259951

(57) **Abstract**

Provided is a blow molding machine (1) including a sound collection device (31) that can detect sound; and one or more arithmetic logic devices (33). At least one of the one or more arithmetic logic devices (33) is configured to execute a product determination function for determining a presence or absence of a product defect, based on an electric signal associated with the sound detected by the sound collection device (31).

## Description

### Technical Field

The present invention relates to a blow molding machine and a method for determining a product defect.

### Background Art

Plastic bottles used for beverage containers and the like are typically obtained by biaxial stretch blow molding (hereinafter simply referred to as blow molding) using a preform as the base material. In order to increase the productivity of plastic bottles, studies have been conducted to prevent molding defects in blow molding. For example, JP 2020-32610 A (Patent Document 1) describes a blow molding device with measures to prevent burrs, which adhere to a mold during molding, from adhering to a product. Also, JP 2016-74219 A (Patent Document 2) describes a beverage filling device that can use a sterilization method of spraying a low-concentration hydrogen peroxide while also preventing molding defects such as whitening, distortion, and molding unevenness.

### Citation List

### Patent Literature

Patent Document 1: JP 2020-32610 A
Patent Document 2: JP 2016-74219 A

### Summary of Invention

### Technical Problem

Although the techniques described in Patent Documents 1 and 2 are intended to prevent the occurrence of molding defects, there is room for further studies into detecting molding defects unable to be prevented and preventing defective products from flowing to the downstream processes. For example, typically, pressure of a pressurized fluid blown into a preform during blow molding is monitored, and a product defect is detected based on the pressure. However, the detection accuracy is insufficient in some cases.

Thus, there is a demand for a blow molding machine and a method for determining a product defect that can detect product defects with higher accuracy than known techniques.

### Solution to Problem

A blow molding machine according to the present invention includes a sound collection device that can detect sound; and one or more arithmetic logic devices. At least one of the one or more arithmetic logic devices is configured to execute a product determination function for determining a presence or absence of a product defect, based on an electric signal associated with the sound detected by the sound collection device.

A method for determining a product defect according to the present invention includes collecting sound emitted from a blow molding machine; and determining a presence or absence of a product defect in the blow molding machine, based on the sound collected in the collecting.

In these configurations, a distinctive sound that is made when a product defect occurs during molding is targeted, and by detecting the sound, product defects can be successfully detected with a higher accuracy than known techniques.

Preferred aspects of the present invention will be described below. However, the scope of the present invention is not limited by the preferred aspects described below.

In the blow molding machine according to an aspect of the present invention, preferably, at least one of the one or more arithmetic logic devices determines that the product defect is present in response to an intensity of a component, which is included in the electric signal, relating to sound at a predetermined frequency exceeding a predetermined threshold.

According to this configuration, since a product defect can be determined using a relatively simple determination condition, the determination condition can be easily adjusted.

In the blow molding machine according to an aspect of the present invention, preferably, the predetermined frequency is selected from 40 to 65 Hz or 5000 to 20000 Hz.

According to this configuration, a burst, which is a defect where a preform has at least partially ruptured during deformation or after deformation, can be detected with high accuracy, based on a sound of a distinctive frequency that is emitted when a burst occurs.

The blow molding machine according to an aspect of the present invention preferably further includes a pressure sensor that can detect pressure of a pressurized fluid to be supplied to expand a product. Preferably, at least one of the one or more arithmetic logic devices is further configured to execute an instrument determination function for determining a presence or absence of an instrument abnormality, based on an electric signal associated with the pressure detected by the pressure sensor.

According to this configuration, since the instrument determination function can be executed in addition to the product determination function, product quality can be maintained at a higher level.

In the blow molding machine according to an aspect of the present invention, preferably, at least one of the one or more arithmetic logic devices is configured to notify a worker of the product defect, in response to determining that the product defect is present.

According to this configuration, a worker can learn that a product defect has occurred at any early stage.

Further features and advantages of the present invention will become more apparent from the following description of exemplary and non-limiting embodiments with reference to the drawings.

### Brief Description of Drawings

FIG. 1 is a block diagram illustrating a configuration of a blow molding machine.
FIG. 2 is a schematic plan view of a rotary device.

### Description of Embodiments

Embodiments of a blow molding machine and a method for determining a product defect according to the present invention will be described with reference to the drawings. Hereinafter, an example in which a blow molding machine according to the present invention is applied to a blow molding machine 1 that can mold a plastic bottle (hereinafter simply referred to as a "bottle") will be described.

In the present embodiment, the molded bottle is made of polyethylene terephthalate, is colorless, and is transparent. The bottle is obtained by biaxial stretch blow molding using a preform made of polyethylene terephthalate as a base material. The volume of the bottle is not particularly limited and may be a commonly distributed volume ranging from approximately 200 mL to 2 L, such as 280 mL, 350 mL, or 500 mL. The bottle is used as a container for liquid products such as beverages including soft drinks (carbonated drinks, fruit drinks, coffee drinks, tea drinks, mineral water, soymilk, vegetable drinks, sports drinks, cocoa drinks, and the like), alcoholic drinks, and milk drinks; liquid foods such as soups; liquid seasonings such as sauces and soy sauce; and the like, for example. The material that the bottle is made of is not limited to polyethylene terephthalate and can be polyethylene, polypropylene, or the like, for example.

### Configuration of Blow Molding Machine

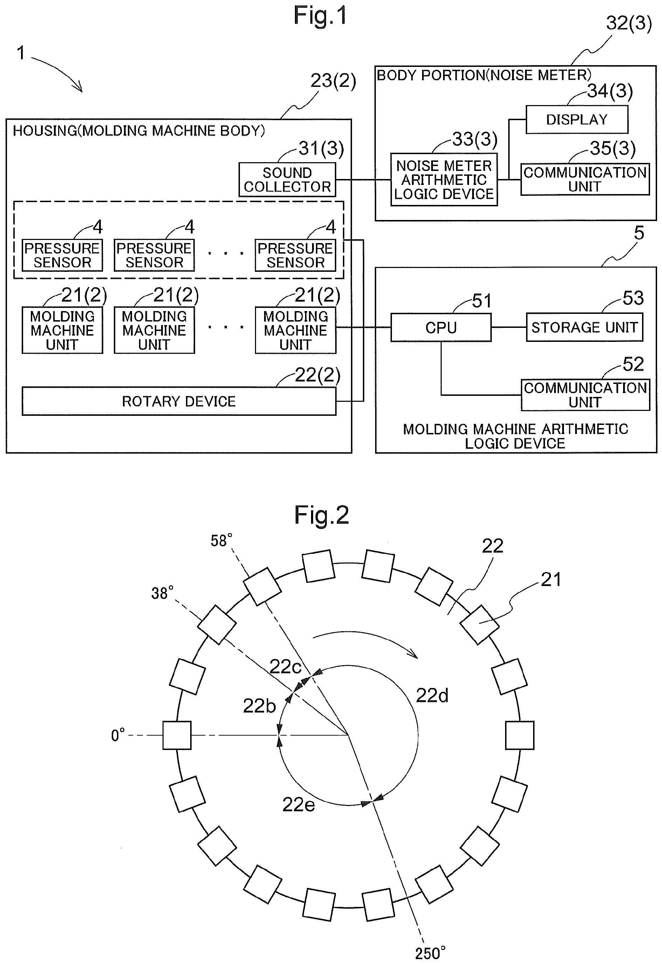

The blow molding machine 1 according to the present embodiment includes a molding machine body 2, a noise meter 3, a pressure sensor 4, and a molding machine arithmetic logic device 5 (an example of an arithmetic logic device) (FIG. 1).

The molding machine body 2 includes a plurality of molding machine units 21 and a rotary device 22, and the molding machine units 21 and the rotary device 22 are housed in a housing 23. In the present embodiment, eighteen molding machine units 21 are arranged at equal intervals along the circumference of the rotary device 22. Each molding machine unit 21 repeats a cycle of sequentially performing preform receiving, primary stretching, secondary stretching, and bottle discharging while moving in accordance with the rotational motion of the rotary device 22. Each molding machine unit 21 is provided with a mold, a stretching rod, a temperature control device, a compressed air pipe, and the like, which are all known components of a biaxial stretch blow molding device and thus will not be described in detail here.

FIG. 2 is a schematic plan view of the rotary device 22, with the zoning of the rotary device 22 also illustrated in the diagram. The range of each zone is specified by an angle range along the rotation direction (clockwise in the present embodiment) of the rotary device 22 with a point 22a where the preform is delivered from the previous process being set as 0°. In addition, the molding machine body 2 is configured to output, to the molding machine arithmetic logic device 5, an electric signal representing a value of an angle based on the point 22a being 0° for the current position of each molding machine unit 21.

A region at an angle from 0 to 38° is a preheating region 22b. The preform received by the molding machine unit 21 at the point 22a is heated while being conveyed through the preheating region 22b. The output of the temperature control device is feedback-controlled such that a temperature setting value for each unit (for example, preform 110°C, mold 74°C) is realized.

A region at an angle from 38 to 58° is a primary stretching region 22c. In the primary stretching region 22c, the stretching rod is advanced while compressed air is blown into the preheated preform to stretch the preform in the longitudinal direction.

A region at an angle from 58 to 250° is a secondary stretching region 22d. In the secondary stretching region 22d, compressed air is blown into the longitudinally stretched preform to stretch (expand) the preform in the lateral direction. The external shape of the preform laterally stretched is regulated by the mold to form the shape of a bottle, and thus a bottle is obtained. In the latter half portion (225 to 250°) of the secondary stretching region 22d, a part of the blown compressed air is discharged. The discharged compressed air may be collected.

A region at an angle from 250 to 360° (0°) is a discharging region 22e. In the discharging region 22e, the obtained bottles are discharged to the next process. Furthermore, in the discharging region 22e, the molding machine unit 21 that has discharged a bottle is put in a state ready to receive the next preform before returning to the point 22a (0°). Note that in the discharging region 22e, air is also collected.

The noise meter 3 includes a sound collector 31 (an example of a sound collection device) and a body portion 32. The body portion 32 is provided with a noise meter arithmetic logic device 33 (an example of an arithmetic logic device), a display 34, and a communication unit 35.

The sound collector 31 is a device configured to detect sound, convert the detected sound into an electric signal, and output the electric signal. The sound collector 31 is attached to the inside of the housing 23 of the molding machine body 2. The body portion 32 is attached to the outside of the housing 23 of the molding machine body 2. The sound collector 31 and the body portion 32 are connected by wire, and an electric signal output from the sound collector 31 is input to the noise meter arithmetic logic device 33.

The noise meter arithmetic logic device 33 can execute a product determination function for determining the presence or absence of a product defect, based on the electric signal associated with the sound detected by the sound collector 31. The noise meter arithmetic logic device 33 can communicate with other devices via the communication unit 35. An example of a communication partner of the noise meter arithmetic logic device 33 is an empty bottle inspection machine (not illustrated) provided downstream of the blow molding machine 1.

The display 34 displays the operation status of the noise meter 3, information relating to the detected sound, and the like.

The pressure sensor 4 is configured to detect the pressure of the air (an example of a pressurized fluid) to be supplied to the molding machine body 2. A pipe for supplying air to the molding machine body 2 branches to reach each of the eighteen molding machine units 21 inside the molding machine body 2, and eighteen pressure sensors 4 are provided correspondingly thereto. In other words, the pressure of the air supplied to each molding machine unit 21 can be detected. The pressure sensor 4 can convert the detected pressure into an electric signal and output the electric signal. The pressure sensor 4 can be implemented as a strain gauge, for example.

The molding machine arithmetic logic device 5 includes a CPU 51, a communication unit 52, and a storage unit 53. The electric signal output from the pressure sensor 4 and the electric signal indicating the current position angle of each molding machine unit 21 output from the molding machine body 2 are input to the CPU 51. The CPU 51 is configured to execute a control program stored in the storage unit 53. By executing the control program, the CPU 51 can execute an instrument determination function for determining the presence or absence of an instrument abnormality.

The molding machine arithmetic logic device 5 can communicate with other devices via the communication unit 52. For example, the molding machine arithmetic logic device 5 can transmit various types of warnings to a terminal owned by a worker.

### Product Determination Function

The product determination function is a function for determining the presence or absence of a product defect based on an electric signal output from the noise meter 3.

A typical product defect that can be detected by the product determination function is a burst. A burst refers to a phenomenon in which, when a preform is deformed into a bottle shape by pressurizing the inside of the preform in the molding machine body 2, the preform is at least partially ruptured during or after the deformation. When the burst occurs, the wall surface of the bottle may be torn or perforated, so that the function of the bottle is impaired. Accordingly, filling the bottle having a burst with a liquid product is to be avoided.

As described above, since the burst is a phenomenon accompanied by a rupture of the preform, a rupturing sound is generated when the burst occurs. Thus, in the present embodiment, the noise meter arithmetic logic device 33 distinguishes, of the electric signal output from the sound collector 31, between a portion based on environmental sounds (for example, the operating sounds of devices installed near the sound collector 31) that are present irrespective of whether a burst has occurred and a portion based on a rupturing sound at the time of a burst and determines that a product defect (burst) has occurred when an electric signal based on a rupturing sound is input into the noise meter arithmetic logic device 33.

More specifically, when the intensity of a component, which is included in the input electric signal, relating to sound at a predetermined frequency is greater than a threshold, the noise meter arithmetic logic device 33 determines that a product defect (burst) has occurred.

As an example, the predetermined frequency is preferably set to from 40 to 65 Hz. In this frequency band, since the intensity of both the environmental sound and the rupturing sound are sufficiently great, it is easy to obtain an electric signal of an amount and quality required for determination.

Furthermore, as another example, the predetermined frequency is preferably set to from 5000 to 20000 Hz. In this frequency band, although the intensity of the rupturing sound itself is less than the range from 40 to 65 Hz described above, the difference in intensity between the environmental sound and the rupturing sound is large, and thus it is easy to identify the presence or absence of a rupturing sound. In this example, the predetermined frequency is more preferably 6000 Hz or higher and even more preferably 7000 Hz or higher. In addition, the predetermined frequency is more preferably 10000 Hz or less and even more preferably 8000 Hz or less. Accordingly, the predetermined frequency can be from 7000 to 8200 Hz, for example.

When the noise meter arithmetic logic device 33 determines that there is a product defect (burst) through the product determination function, the noise meter arithmetic logic device 33 transmits a notification signal relating to the burst to the empty bottle inspection machine via the communication unit 35. The empty bottle inspection machine, having received the notification signal, further notifies a production management system (not illustrated) of the burst. Furthermore, the production management system transmits a notification signal relating to the burst to a terminal owned by a worker. The worker who receives the notification inspects the bottles molded in the molding machine body 2 within a time period including the burst detection time, identifies the bottle with a burst, and rejects it.

### Instrument Determination Function

The instrument determination function is a function for determining the presence or absence of an instrument abnormality based on an electric signal output from the pressure sensor 4.

When the molding machine unit 21 is located in the primary stretching region 22c and the secondary stretching region 22d of the rotary device 22, compressed air is supplied into the preform to stretch the preform. Here, the specified pressure of the supplied compressed air is different between the primary stretching region 22c and the secondary stretching region 22d.

As described above, the pressure sensor 4 is provided for each molding machine unit 21 (for a total of eighteen), allowing the pressure of the air supplied to each molding machine unit 21 to be detected. Furthermore, an electric signal representing the current position angle of each molding machine unit 21 is input into the molding machine arithmetic logic device 5.

With the configuration described above, the molding machine arithmetic logic device 5 can create a plot of the current position angle (horizontal axis) and the pressure of the supplied air (vertical axis) for each molding machine unit 21. Then, a specified range of pressure is set for each of the primary stretching region 22c and the secondary stretching region 22d, and the arithmetic logic device determines that an instrument abnormality has occurred when the pressure deviates from the specified range. A deviation from the specified range can be caused by a valve defect (leakage or clogging), for example.

For example, the specified range of pressure in the primary stretching region 22c is set to from 1.5 bar to 4.0 bar. Accordingly, when the current position angle of the molding machine unit 21 is from 38 to 58° and the pressure of the compressed air is less than 1.5 bar or greater than 4.0 bar, the molding machine arithmetic logic device 5 (CPU 51) determines that an instrument abnormality has occurred.

Similarly, the specified range of pressure in the secondary stretching region 22d is set to from 20 bar to 40 bar. Accordingly, when the current position angle of the molding machine unit 21 is from 58 to 225° and the pressure of the compressed air is less than 20 bar or greater than 40 bar, the molding machine arithmetic logic device 5 (CPU 51) determines that an instrument abnormality has occurred.

When the instrument determination function determines that an instrument abnormality such as a valve defect is present, the molding machine arithmetic logic device 5 (CPU 51) transmits a notification signal relating to the instrument abnormality to a terminal owned by a worker via the communication unit 52. Accordingly, the worker can learn of the instrument abnormality at an early stage.

### Other Embodiments

Finally, other embodiments of a blow molding machine and a method for determining a product defect according to the present invention will be described. Note that the configuration described in each of the following embodiments can also be applied in combination with the configuration described in another embodiment as long as no contradiction arises.

In the example configuration of the embodiment described above, the blow molding machine 1 includes the pressure sensors 4, and the molding machine arithmetic logic device 5 is configured to execute the instrument determination function. However, the blow molding machine according to the present invention need not include a pressure sensor.

In the example configuration of the embodiment described above, in the product determination function, when the intensity of the component relating to sound at a predetermined frequency is greater than a threshold, it is determined that a product defect (burst) has occurred. However, in the product determination function of the blow molding machine according to the present invention, the method for determining the presence or absence of a product defect is not limited to the example described above. For example, a method for determining the presence or absence of a product defect based on a waveform of an electric signal output from a noise meter, a method for determining the presence or absence of a product defect based on information obtained through statistical processing of the electric signal, or the like can be employed. Also, even if it is determined that a product defect has occurred when the intensity of the component relating to sound at a predetermined frequency is greater than a threshold as in the example described above, the predetermined frequency is not particularly limited. The predetermined frequency is preferably selected from 40 to 65 Hz or 5000 to 20000 Hz.

In the embodiment described above, the molding machine arithmetic logic device 5 is configured to transmit various types of warnings to a terminal owned by a worker. However, the measures taken when the blow molding machine according to the present invention determines that a product defect is present are not limited to issuing a warning. Other measures that can be taken include stopping the blow molding machine, recording that a product defect has occurred, and the like. Also, a combination of issuing a warning and all of the other measures described can be executed. On the other hand, a configuration in which such measures are not performed can be employed. The presence or absence of measures to be taken when a product defect is detected and the details of the measures can be set as appropriate depending on the environment (the number of workers, the required quality level, the status of other processes, and the like) where the blow molding machine is operating.

In the example configuration of the embodiment described above, the noise meter arithmetic logic device 33 provided in the noise meter 3 can execute the product determination function. However, in the blow molding machine according to the present invention, the position where the arithmetic logic device that can execute the product determination function is provided is not limited. For example, the product determination function may be configured to be executed by an arithmetic logic device provided in the blow molding machine body.

In the example configuration of the embodiment described above, the noise meter arithmetic logic device 33 can execute the product determination function, and the molding machine arithmetic logic device 5 can execute the instrument determination function. However, in a case where the blow molding machine according to the present invention can execute the product determination function and the instrument determination function, the arithmetic logic device that executes both functions may be a single device or a plurality of devices. For example, the product determination function and the instrument determination function may be executed by an arithmetic logic device provided in the blow molding machine body.

In the example configuration of the embodiment described above, the sound collector 31 is attached to the inside of the housing 23 of the molding machine body 2. However, the place where the sound collection device is provided in the blow molding machine according to the present invention is not limited and may be outside of the housing of the molding machine body, for example.

Also, with respect to other configurations, it should be understood that the embodiments described in the present specification are illustrative in all respects and that the scope of the present invention is not limited by them. It will be easily understood by those skilled in the art that appropriate modifications can be made without departing from the spirit of the present invention. Accordingly, other embodiments modified without departing from the spirit of the present invention are naturally included in the scope of the present invention.

### Industrial Applicability

The present invention can be used to detect a burst defect in a blow molding machine, for example.

### Reference Signs List

1: Blow molding machine
2: Molding machine body
21: Molding machine unit
22: Rotary device
22b: Preheating region
22c: Primary stretching region
22d: Secondary stretching region
22e: Discharging region
23: Housing
3: Noise meter
31: Sound collector
32: Body portion
33: Noise meter arithmetic logic device
34: Display
35: Communication unit
4: Pressure sensor
5: Molding machine arithmetic logic device
51: CPU
52: Communication unit
53: Storage unit

## Claims

1. A blow molding machine comprising:
a sound collection device configured to detect sound; and
one or more arithmetic logic devices,
wherein at least one of the one or more arithmetic logic devices is configured to execute a product determination function for determining a presence or absence of a product defect, based on an electric signal associated with the sound detected by the sound collection device.

2. The blow molding machine according to claim 1,
wherein at least one of the one or more arithmetic logic devices determines that the product defect is present, in response to an intensity of a component, which is included in the electric signal, relating to sound at a predetermined frequency exceeding a predetermined threshold.

3. The blow molding machine according to claim 2,
wherein the predetermined frequency is selected from 40 to 65 Hz or 5000 to 20000 Hz.

4. The blow molding machine according to any one of claims 1 to 3, further comprising
a pressure sensor configured to detect pressure of a pressurized fluid to be supplied to expand a product,
wherein at least one of the one or more arithmetic logic devices is further configured to execute an instrument determination function for determining a presence or absence of an instrument abnormality, based on an electric signal associated with the pressure detected by the pressure sensor.

5. The blow molding machine according to any one of claims 1 to 4,
wherein at least one of the one or more arithmetic logic devices is configured to notify a worker of the product defect, in response to determining that the product defect is present.

6. A method for determining a product defect comprising:
collecting sound emitted from a blow molding machine; and
determining a presence or absence of a product defect in the blow molding machine, based on the sound collected in the collecting.
